Europäisches Patentamt

European Patent Office

Office européen des brevets

(11) Publication number: **0 625 343 A2**

# EUROPEAN PATENT APPLICATION

(21) Application number: **94201302.0**

(22) Date of filing: **09.05.94**

(51) Int. Cl.⁵: **A61F 5/441**, A61L 15/00, B32B 27/30

(30) Priority: **11.05.93 US 60188**

(43) Date of publication of application: **23.11.94 Bulletin 94/47**

(84) Designated Contracting States: **AT BE CH DE DK ES FR GB GR IE IT LI LU MC NL PT SE**

(71) Applicant: **Bristol-Myers Squibb Company**
**345 Park Avenue**
**New York, N.Y. 10154 (US)**

(72) Inventor: **Torgalkar, Anil**
**73 Maplestream Road**
**East Windsor, NJ 08520 (US)**
Inventor: **Klem, Leslie**
**202 Fulton Street**
**Trenton, NJ 08611 (US)**
Inventor: **Shah, Kishore**
**568 Cabot Hill Road**
**Bridgewater, NJ 08807 (US)**

(74) Representative: **Mays, Julie**
**Bristol-Myers Squibb Company,**
**Patent Department,**
**Swakeleys House,**
**Milton Road**
**Ickenham, Uxbridge UB10 8NS (GB)**

(54) **Medical products and methods using chloride-free odor barrier materials.**

(57) Methods and devices for medical use such as ostomy and wound healing having excellent chloride-free odor barrier properties are disclosed. It has been found that laminates incorporating ethylene vinyl alcohol (EVOH) copolymers are useful in providing an excellent barrier to odor and moisture without sacrificing softness and noise characteristics important to, for example, ostomy devices. The EVOH barrier is preferably between about 5-12% of the total laminate thickness.

Rank Xerox (UK) Business Services
(3.10/3.09/3.3.4)

The present invention relates to novel methods for providing odor barriers in ostomy devices and is particularly concerned with such methods which utilize non-polyvinylidene chloride-based materials.

There are a number of materials known to have significant gas barrier characteristics which are effective by providing resistance to gas and aroma permeation and transmission. Such materials include polyvinylidene chloride (PVDC), including the vinyl chloride copolymer (PVDC-VC) and methacrylate copolymer (PVDC-MA) thereof, ethylene vinyl alcohol (EVOH) resins, nylon polymers including nylon 66 and nylon 66/6 10 copolymer, polypropylene and polyethylene terpthalate (PET). Also known are barrier composites including, but not limited to, glass-coated polyethylene terphthalate and two or three-component laminates including EVOH or PVDC. These barrier materials are used as packaging films, particularly in the food industry.

PVDC and its copolymers have also been widely used as odor barrier materials in ostomy devices. In fact, PVDC-based barriers are the only materials from the above group which have thus far been found suitable for ostomy use. This is because the performance standards for use of odor barriers in ostomy devices, e.g., colostomy pouches, urostomy devices and the like, are significantly more stringent than for use in the packaging industries. First, as expected, the odors generated in an ostomy setting are of a substantially higher degree than found in, for example, the food industry. Also, the gas transmission rate of most of these non-PVDC barrier materials is adversely affected (i.e., increased) in high humidity environments such as the ostomy setting. Another medical area requiring odor barriers which can withstand high humidity is the wound care art where wounds of heavy exudate volume are a problem.

Although it is known that barrier materials susceptible to humidity can be coextruded with polymers having low water vapor transmission rates, it must be noted that the barrier laminate is subjected to the high humidity in the ostomy and/or wound setting for a sustained period of time. If the barrier films or laminates are made thicker to resist the adverse effects of moisture the medical device becomes unacceptably noisy and suffers from loss of softness and pliability.

Although PVDC-based barrier materials have been able to meet the stringent standards for ostomy use, it may prove desirable to have a "non-chloride-containing" alternative for these and other medical products.

In accordance with the present invention methods of providing an odor barrier for medical products and the medical products including such a barrier are disclosed. It has been found that medical products useful in areas such as ostomy and wound care where high humidity and a high degree of odor are problematic are unexpectedly provided with excellent odor barrier, softness and noise characeristics without the use of chloride containing materials. The methods and products of this invention employ ethylene vinyl alcohol (EVOH) copolymers wherein the vinyl alcohol content is between about 45 and 65 mole percent of the copolymer. Such EVOH copolymers are preferably used in a laminate comprising the ethylene vinyl alcohol copolymer interposed two polymer films.

These present inventors have found that ethylene vinyl alcohol copolymer-based laminates are extremely useful for use in medical devices, e.g., ostomy devices or wound dressings, where a high degree of odor and high humidity must be contained. Laminates in accordance with the present invention are able to provide an excellent barrier to the extreme odors of human waste or wound exudate without being adversely affected by sustained high humidity. Preferred laminates which provide a sustained odor barrier for human waste or wound exudate in high humidity are those wherein the EVOH copolymer makes up at least 2 percent of the laminate thickness. More preferably, for laminates ideally suited for ostomy use which maintain a high degree of softness and low degree of noise, the EVOH accounts for 2-12 percent of the laminate thickness. Most preferred are laminates wherein the EVOH is 2-10 percent of the laminate thickness.

Alternatively, the EVOH barrier layer may also contain additives, i.e., plasticizers, etc., or may be blended with other polymeric materials, e.g., EVA and the like. When additives are desired, the barrier thickness needs to be adjusted (increased) so that an amount of EVOH equivalent to that in the 2-12% thickness barrier is still employed. Accordingly, barrier thicknesses when EVOH includes additives may be up to about 20 percent of the total laminate thickness.

Ethylene vinyl alcohol copolymer resins suitable for use herein typically have a vinyl alcohol content between about 45 and 65 mole percent of the EVOH copolymer. More preferably, the vinyl alcohol content is between about 50 and about 55 mole percent. Most preferred is when the EVOH copolymer is about 52 mole percent vinyl alcohol.

Laminates useful herein may be of two, three or more components wherein a desired polymer to be coextruded with the EVOH copolymer material is the inside portion of the laminate in contact with the waste in the ostomy device or closest to the exudating wound in the dressing. Typically, this polymer is chosen for its lower water vapor transmission rates so as to protect the odor barrier properties of the EVOH. Preferred laminates for use herein include the EVOH material sandwiched between two polymer films (typically by coextrusion), which films may be the same or different polymer and/or thickness. Most preferred are those laminates comprising EVOH interposed two equal thickness films of the same polymer, typically a copolymer of EVA. Tie layers can be incorporated into the laminate as required to improve adhesion of the polymer layer(s) to the EVOH barier layer. Typical tie layer materials include copolymers of ethylene vinyl acetate (EVA), ethylene vinyl alcohol (EVOH), ethylene acrylic acid (EAA) and the like.

As mentioned above, any desired polymer or polymers may be used for the outer layers of the laminate, i.e., other than the EVOH layer. Suitable polymers include, but are not limited to, high density polyethylene (HDPE), low density polyethylene (LDPE), EVA, ethylene methacrylate (EMA), nylon polymers and copolymers, polyesters, polypropylene, thermoplastic polyurethanes, thermoplastic elastomers, polymer alloys, natural and synthetic rubbers and copolymers thereof. Preferred polymers for use herein, based upon their physical (softness, quiet) and water vapor transmission characteristics include EVA, EMA, LDPE, thermoplastic polyurethanes, thermoplastic elastomers, polymer alloys, natural and synthetic rubbers and copolymers thereof.

Preferred EVOH-containing laminates in accordance with the present methods and devices are those wherein the EVOH layer accounts for about 2-10 percent of the laminate thickness and the polymer sandwiching layers make up about 45 to 49 percent each of the laminate thickness. Most preferred are the above laminates where the sandwiching layers are EVA.

The total laminate thickness is chosen according to the desired application and can be any convenient thickness. Typical thicknesses for ostomy products are from about 50 to about 125 microns. Typical urostomy laminates are about 100 microns in thickness. Other typical ostomy laminates are about 75 microns in thickness. Laminates for use in wound care or other medical settings can be of any desired thickness, e.g., 25 to 200 microns, or as desired.

The ethylene vinyl alcohol resins for preparing the barrier films of the present invention are known and are commercially available, e.g., from EVAL Company of America or DuPont. Films made from such resins are also commercially available, for example, from American National Can Company and Consolidated Thermoplastics Company. Similarly, the laminates herein employ known, commercially available polymer films for the other layers. Laminates of this invention are prepared using methodology known to those skilled in the polymer film art. For example, the polymer resins for each of the desired layers of the laminate are melted and coextruded through an annular type multi-layer die to distribute the liquid polymers evenly about the annulus. Upon emerging from the circular die gap the multi-layer molten/film is drawn vertically upward and simultaneously blown outward by air pressure within a so-formed bubble of the molten material. The melt is cooled and drawn by blowing air on the exterior of the bubble via an air ring. The film is then collapsed and slit prior to winding.

The present invention is further described by the following real examples, however, it is to be understood that this invention should not be limited to the details described therein.

Example 1

Five different EVOH barrier films (EVA/EVOH/ EVA laminates) were fabricated into various ostomy pouch devices and tested against similar devices of the prior art having polyvinylidene chloride barriers (EVA/PVDC/EVA laminates) to compare odor barrier characteristics. Five devices were fabricated for each film/pouch type and were compared to five prior art PVDC barriers of the same thickness/pouch type.

Into each pouch was loaded 25 grams of chicken/turkey manure and 250 ml of water. Each pouch was placed into a separate mason jar and sealed. Periodically the jars were checked for mal-odor by averaging the "no-odor" data (i.e., time just before first odor) and the "first odor" data. The results, demonstrating comparable odor barrier characteristics for the non chloride-containing EVOH devices are set forth in Table 1. The odor barrier results are expressed as a percentage of the control devices. Samples 1, 3 and 4 were compared to devices made of MF374 3 mil PVDC laminate from W. R. Grace. Samples 2 and 5 were compared to devices made of MF400 4 mil laminate from W. R. Grace. Samples 4 and 5 incorporated a plasticizer into the EVOH barrier material and the thickness of these films was increased accordingly.

TABLE 1

| Sample # | Film (EVA/EVOH/EVA) | Pouch Type | Total Film Thickness (mils) | EVOH barrier Thickness (mils) | Barrier % of Thickness | % Odor Barrier Compared to PVDC Control |
|---|---|---|---|---|---|---|
| 1 | 52% vinyl alcohol in EVOH barrier[1] | Sur-Fit® Drainable[3] | 2.87 | 0.22 | 7.7 | 85% |
| 2 | 52% vinyl alcohol in EVOH barrier[1] | Sur-Fit® Urostomy[3] | 3.86 | 0.21 | 5.4 | 89% |
| 3 | 52% vinyl alcohol in EVOH barrier[1] | Active Life[3] | 3.15 | 0.20 | 6.35 | 106% |
| 4 | 56% vinyl alcohol in EVOH barrier[2] | Sur-Fit® Drainable 12" | 3.0 | 0.5 | 16.6[4] | 99% |
| 5 | 58% vinyl alcohol in EVOH barrier[2] | Sur-Fit® Drainable 12" | 3.95 | 0.53 | 13.4[4] | 97% |

[1]Commercially available-American National Can Co.
[2]Commercially available-Consolidated Thermoplastics
[3]Ostomy Products from ConvaTec, Subsidiary of Bristol-Myers Squibb
[4]Includes plasticizer in EVOH barrier

Example 2

Using ASTM procedures, the film properties of EVA/EVOH/EVA 4 mil laminate films (similar to sample 2 in Example 1, above) were obtained and compared to properties of the 4 mil PVDC-containing control film of Example 1. The properties are summarized in Table 2. These films are ideally suited for urostomy devices. The values for tensile strength, Young's modulus, elongation to break, yield stress and energy to break are average of 65 samples.

### TABLE 2

#### FILM PHYSICAL PROPERTIES

| Property | | Sample 2 - type Laminate | PVDC-containing CONTROL |
|---|---|---|---|
| Thickness (MIL) | | 4.0 | 4.0 |
| Tensile Strength (MPA) | MD | 15.6 ± 0.8 | 29.5 ± 4.6 |
| | TD | 14.0 ± 0.6 | 28.6 ± 0.9 |
| Young's Modulus (MPA) | MD | 117.7 ± 5.9 | 172.3 ± 8.5 |
| | TD | 116.5 ± 5.9 | 159.6 ± 6.7 |
| Elongation @ Break (%) | MD | 448.0 ± 26.7 | 460.0 ± 60.0 |
| | TD | 555.7 ± 53.3 | 586.6 ± 19.7 |
| Yield Stress (MPA) | MD | 7.4 ± 0.3 | 12.0 ± 0.6 |
| | TD | 7.3 ± 0.1 | 9.2 ± 0.1 |
| Energy to Break (J) | MD | 2.4 ± 0.2 | 4.3 ± 1.0 |
| | TD | 2.9 ± 0.3 | 4.3 ± 0.2 |
| Oxygen Transmission (cc/M$^2$/day) @ 100% RH, RT | | 22.3 | 30 - 50 |
| MVTR* (G/M$^2$/day) | | 22.2 | 3 - 5 |

*Moisture Vapor Transmission Rate

Example 3

Using ASTM procedures, the film properties of EVA/EVOH/EVA 3 mil laminate films (similar to sample 1 in Example 1, above) were obtained and compared to properties of the 4 mil PVDC-containing control film of Example 1. The properties are summarized in Table 3. These films are ideally suited for urostomy, iliostomy devices.

As above, the data for the first five tests are averages of 65 samples.

TABLE 3

FILM PHYSICAL PROPERTIES

| Property | | | Sample 1 - type Laminate | PVDC-containing CONTROL |
|---|---|---|---|---|
| Thickness (MIL) | | | 3.0 | 3.0 |
| Tensile Strength (MPA) | | MD | 20.9 ± 1.8 | 22.3 ± 1.1 |
| | | TD | 15.7 ± 1.2 | 18.2 ± 0.5 |
| Young's Modulus (MPA) | | MD | 153.3 ± 20.3 | 142.3 ± 6.8 |
| | | TD | 147.1 ± 20.3 | 144.3 ± 7.6 |
| Elongation @ Break (%) | | MD | 438.0 ± 23.8 | 398.5 ± 13.5 |
| | | TD | 600.6 ± 50.9 | 529.2 ± 13.2 |
| Yield Stress (MPA) | | MD | 9.1 ± 0.4 | 9.7 ± 0.3 |
| | | TD | 8.3 ± 0.2 | 7.8 ± 0.2 |
| Energy to Break (J) | | MD | 2.3 ± 0.3 | 2.1 ± 0.1 |
| | | TD | 2.6 ± 0.3 | 2.0 ± 0.1 |
| Oxygen Transmission (cc/M$^2$/day) @ 100% RH, RT | | | 26.2 ± 10.3 | 30 - 50 |
| MVTR* (G/M$^2$/day) | | | 21.4 ± 4.0 | 3 - 5 |

*Moisture Vapor Transmission Rate

## Claims

1. A method for providing an odor barrier in a medical device which method comprises using an ethylene vinyl alcohol copolymer as a barrier material, wherein the vinyl alcohol is present in an amount of from about 45 to about 65 mole percent of the polymer.

2. The method of claim 1 wherein the ethylene vinyl alcohol copolymer is in the form of a film.

3. The method of claim 2 wherein the ethylene vinyl alcohol copolymer forms a laminate with one or more polymer films and wherein the ethylene vinyl alcohol copolymer makes up at least 2 percent of the thickness of the laminate.

4. The method of claim 3 wherein the ethylene vinyl alcohol copolymer makes up from 2 to about 12 percent of the thickness of the laminate.

5. The method of claim 3 wherein said laminate comprises said ethylene vinyl alcohol copolymer film interposed two polymer films.

6. The method of claim 5 wherein said polymer films are of the same polymer.

7. The method of claim 5 wherein said polymer films are of equal thickness.

8. The method of claim 1 wherein said ethylene vinyl alcohol copolymer has a vinyl alcohol content in the range of from about 50 to about 55 mole percent of the copolymer.

9. The method of claim 8 wherein said ethylene vinyl alcohol copolymer has a vinyl alcohol content of about 52 mole percent of the copolymer.

10. The method of claim 3 wherein the polymer films are selected from high density polyethylene (HDPE), low density polyethylene (LDPE), EVA, ethylene methacrylate (EMA), nylon polymers and copolymers, polyesters, polypropylene, thermoplastic polyurethanes, thermoplastic elastomers, polymer alloys, natural and synthetic rubbers and copolymers thereof.

11. The method of claim 10 wherein the polymer films are selected from EVA, EMA, LDPE, thermoplastic polyurethanes, thermoplastic elastomers, polymer alloys, natural and synthetic rubbers and copolymers thereof.

12. The method of claim 3 wherein said laminate includes tie layers selected from copolymers of EVA, EVOH and EAA.

13. The method of claim 3 wherein said laminate is between about 50 and 200 microns in thickness.

14. The method of claim 1 wherein said barrier consists of a laminate comprising
a) an ethylene vinyl alcohol copolymer barrier layer including about 52 mole percent of vinyl alcohol; and
b) first and second polymer layers overlying the surfaces of said barrier layer, said polymer layers selected from the group EVA, EMA, LDPE, thermoplastic polyurethanes, thermoplastic elastomers, polymer alloys, natural and synthetic rubbers and copolymers thereof;
wherein said barrier layer accounts for about 2 to 10 percent of the laminate thickness.

15. The method of claim 1 wherein said EVOH barrier layer further includes one or more additives and wherein the thickness of said layer is increased such that an equivalent amount of EVOH is employed.

16. A medical device suitable for use in areas of high humidity and odor from mammalian waste or exudate, which device includes a film of pliable material for the containment of said waste or exudate and the odors therefrom, the improvement wherein said film includes an odor barrier of an ethylene vinyl alcohol copolymer, wherein the vinyl alcohol content is from about 45 to about 65 mole percent of the copolymer.

17. The device of claim 16 being an ostomy device.

8

18. The device of claim 17 wherein the ethylene vinyl alcohol copolymer forms a laminate with one or more polymer films and wherein the ethylene vinyl alcohol copolymer makes up at least 2 percent of the thickness of the laminate.

19. The device of claim 18 wherein the ethylene vinyl alcohol copolymer makes up from 2 to about 12 percent of the thickness of the laminate.

20. The device of claim 18 wherein said laminate comprises said ethylene vinyl alcohol copolymer film interposed two polymer films.

21. The device of claim 20 wherein said polymer films are of the same polymer.

22. The device of claim 20 wherein said polymer films are of equal thickness.

23. The device of claim 1 wherein said ethylene vinyl alcohol copolymer has a vinyl alcohol content in the range of from about 50 to about 55 mole percent of the copolymer.

24. The device of claim 23 wherein said etheylene vinyl alcohol copolymer has a vinyl alcohol content of about 52 mole percent of the copolymer.

25. The device of claim 18 wherein the polymer films are selected from high density polyethylene (HDPE), low density polyethylene (LDPE), EVA, ethylene methacrylate (EMA), nylon polymers and copolymers, polyesters, polypropylene, thermoplastic polyurethanes, thermoplastic elastomers, polymer alloys, natural and synthetic rubbers and copolymers thereof.

26. The device of claim 25 wherein the polymer films are selected from EVA, EMA, LDPE, thermoplastic polyurethanes, thermoplastic elastomers, polymer alloys, natural and synthetic rubbers and copolymers thereof.

27. The device of claim 16 wherein said laminate includes tie layers selected from copolymers of EVA, EVOH and EAA.

28. The device of claim 16 wherein said laminate is between about 50 mole and 200 microns in thickness.

29. The device of claim 16 wherein said barrier consists of a laminate comprising
a) an ethylene vinyl alcohol copolymer barrier layer including about 52 mole percent of vinyl alcohol; and
b) first and second polymer layers overlying the surfaces of said barrier layer, said polymer layers selected from the group EVA, EMA, LDPE, thermoplastic polyurethanes, thermoplastic elastomers, polymer alloys, natural and synthetic rubbers and copolymers thereof;
wherein said barrier layer accounts for about 2 to 10 percent of the laminate thickness.

30. The device of claim 16 wherein said EVOH barrier layer further includes one or more additives and wherein the thickness of said layer is increased such that an equivalent amount of EVOH is employed.